Europäisches Patentamt

European Patent Office

Office européen des brevets

(19)

(11) Veröffentlichungsnummer: **0 133 690**
**B1**

(12)    # EUROPÄISCHE PATENTSCHRIFT

(45) Veröffentlichungstag der Patentschrift:
02.07.86

(21) Anmeldenummer: 84109208.3

(22) Anmeldetag: 03.08.84

(51) Int. Cl.⁴: **C 07 C 21/06,** C 07 C 17/34

(54) **Verfahren zur thermischen Spaltung von 1,2-Dichlorethan.**

(30) Priorität: 09.08.83 DE 3328691

(43) Veröffentlichungstag der Anmeldung:
06.03.85 Patentblatt 85/10

(45) Bekanntmachung des Hinweises auf die Patenterteilung:
02.07.86 Patentblatt 86/27

(84) Benannte Vertragsstaaten:
**BE DE FR GB IT NL**

(56) Entgegenhaltungen:
GB-A-1 494 797

(73) Patentinhaber: **HOECHST AKTIENGESELLSCHAFT,
Postfach 80 03 20, D-6230 Frankfurt am Main 80
(DE)**

(72) Erfinder: **Felix, Bernd, Dr., Rehdorf 52, D-8269
Burgkirchen (DE)**
Erfinder: **Fröhlich, Walter, Dr.,
Kampenwandstrasse 11, D-8269 Burgkirchen (DE)**
Erfinder: **Katzenberger, Heiner, Burgfrieden 8,
D-8263 Burghausen (DE)**

0 133 690

**Beschreibung**

Die Erfindung betrifft ein Verfahren zur thermischen Spaltung von 1,2-Dichlorethan in Gegenwart von bestimmten, die Spaltreaktion beschleunigenden Verbindungen.

Die thermische Spaltung von 1,2-Dichlorethan zu Chlorwasserstoff und Vinylchlorid wird in großem Maße technisch durchgeführt. Das Verfahren ist beispielsweise beschrieben in Ullmanns Encyklopädie der technischen Chemie, 4. Auflage (1975), Band 9, Seiten 447 und 448. Hiernach wird die Spaltung bei 500 bis 550 °C vorgenommen, wobei Umsätze von etwa 50 bis 60 % erzielt werden. Zur Erhöhung des Umsatzes beziehungsweise zum Herabsetzen der Spalttemperatur können sogenannte Initiatoren, beispielsweise in Mengen von 0,2 bis 0,5 %, zugegeben werden. Als solche Initiatoren beziehungsweise Katalysatoren sind Chlor, Sauerstoff, Iod oder Tetrachlorkohlenstoff genannt, es wird jedoch festgestellt, daß sich durch den Zusatz solcher Initiatoren auch der Anfall an Nebenprodukten erhöht, weshalb man in der Technik auf eine Zugabe verzichtet. Aus der gleichen Literaturstelle ist ersichtlich, daß 1,2-Dichlorpropan einen stark inhibierenden, das heißt nachteiligen Effekt auf die thermische Dehydrochlorierung von 1,2-Dichlorethan ausübt.

Aus der DE-AS 12 10 800 ist bekannt, Vinylchlorid durch thermische Spaltung von Dichlorethan bei Drücken zwischen 3 und 20 ata (0,29 bis 1,96 MPa) durch Erhitzen auf Temperaturen zwischen 500 und 620 °C in Gegenwart von 0,5 bis 2 Gew.-% Halogen oder halogenabspaltenden Verbindungen herzustellen. Unter halogenabspaltenden Verbindungen werden Sulfurylchlorid, Thionylchlorid, Hexachlorethan, Tetrachlorkohlenstoff und Perchlorethylen verstanden. Den Chlorverbindungen wird der Vorzug gegeben, Chlorkohlenwasserstoffe mit mehr als 2 Kohlenstoffatomen soll man jedoch wegen der auftretenden Nebenprodukte vermeiden.

Es ist ferner aus der DE-OS 19 53 240 ein Verfahren zur Herstellung von Vinylchlorid durch Dehydrochlorierung von Dichlorethan unter Erhitzen in Gegenwart eines Initiators bekannt, wobei man bei Temperaturen von 250 bis 450 °C unter einem Druck von 2 bis 30 at (0,19 bis 2,94 MPa)in Gegenwart von 0,2 bis 0,9 Gew.-%, bezogen auf eingesetztes Dichlorethan, von einem Initiator arbeitet. Als solche Initiatoren sind angegeben Chlor, Sauerstoff, Nitrosylchlorid oder Hexachlorethan.

Weiterhin ist aus der japanischen Patentanmeldung Sho 42-22921, veröffentlicht am 08.11.67, bekannt, als Initiatoren für die Hitzezersetzung von 1,2-Dichlor-ethan bei einem Druck von 20 bis 35 atm (2,02 bis 3,64 MPa) und einer Reaktionstemperatur von 450 bis 650 °C 0,005 bis 1 Mol-% einer Verbindung der allgemeinen Formel $CX_3NO_2$ (worin X ein H, Cl, Br, $CH_3$ oder $NO_2$ darstellt) zu verwenden.

Bromtrihalogenmethane, beispielsweise $CCl_3Br$ sowie 1,2-Dibromethane sind als Initiatoren für die thermische Zersetzung von 1,2-Dichlorethan bekannt aus den japanischen Patentschriften Sho-40-28779 und Sho 55-68834.

Brom und bromhaltige Verbindungen haben gegenüber Clor und chlorhaltige Verbindungen generell den Nachteil, daß sie bei den hohen zur Zerzetzung des 1,2-Dichlorethans notwendigen Temperaturen erhebliche Korrosionsprobleme mit sich bringen, sie sind außerdem teurer als Chlorverbindungen. Sulfurychlorid und Thionylchlorid benötigen einen zuzätzlichen Aufarbeitungsaufwand, um das gebildete Schwefeldioxid zu entfernen. Das gleiche gilt für Nitrosylchlorid und die nitrogruppenhaltigen Methane der Formel $CX_3NO_2$, die ebenfalls einen erhöhten Aufarbeitungsaufwand benötigen, um die enstehenden Stickoxide zu entfernen, es können hier überdies noch Abgasprobleme auftreten.

Entgegen den Angaben der DE-AS 12 10 800, wonach Chlorkohlenwasserstoffe mit mehr als zwei Kohlenstoffatomen wegen auftretender Nebenprodukte zu vermeiden sind, wurde gefunden, daß bestimmte Chlorkohlenwasserstoffe mit drei Kohlenstoffatomen sehr gute Initiatoren für die thermische Zersetzung von 1,2-Dichlorethan sind, von denen einige in ihrer Wirkung die bisher im Stand der Technik genannten chlorbeziehungsweise nitrogruppenhaltigen Initiatoren übertreffen. Auf gleichen Umsatz des 1,2-Di-chlorethans angewendet ergeben sie weniger unerwünschte Nebenprodukte als beispielsweise bei Zusatz eines bekannten Chlorkohlenwasserstoff-Initiators mit zwei Kohlenstoffatomen oder ohne jeglichen Initiatorzusatz. Die gute Wirkung bestimmter Chlorkohlenwasserstoffe mit drei Kohlenstoffatomen war überraschend, da andere Chlorkohlenwasserstoffe mit drei Kohlenstoffatomen, zum Beispiel 1,2-Dichlorpropan, umgekehrt als Inhibitor auf die thermische Zersetzung von 1,2-Dichlorethan wirken (siehe Ullmann loc. cit.).

Aufgabe der vorliegenden Erfindung ist es, ein Verfahren zur Verfügung zu stellen, das es ermöglicht, die Spalttemperatur bei der thermischen Spaltung von 1,2-Dichlor-ethan herabzusetzen und weniger unerwünschte Nebenprodukte beider Spaltung zu erzeugen.

Diese Aufgabe wird gelöst durch ein Verfahren zur thermischen Spaltung von 1,2-Dichlorethan zu Vinylchlorid bei 300 bis 600 °C, unter Normaldruck oder erhöhtem Druck, in Gegenwart mindestens einer die Spaltreaktion beschleunigenden Verbindung, welches dadurch gekennzeichnet ist, daß als solche Verbindung(en) verwendet werden 0,001 bis 5 Gew.-%, bezogen auf eingesetztes 1,2-Dichlorethan von Trichloracetylchlorid oder einer Verbindung, die 3 Kohlenstoffatome, mindestens 6 Chloratome, 0 oder 1 Sauerstoffatom und, je Kohlenstoffatom an dieses gebunden, 0 oder 1 Wasserstoffatom enthält.

Neben Trichloracetylchlorid geeignete Verbindungen sind beispielsweise Pentachlorpropion säurechlorid, Hexachloraceton, Hexachlorpropan. Besonders gute Ergebnisse werden mit Hexachlorpropen, Heptachlorpropan und Octachlorpropan erhalten. Es können eine der erfindungsgemäß einzusetzenden Verbindungen oder eine Mischung von mehreren solcher Verbindungen sowie auch Mischungen der erfindungsgemäß einzusetzenden Verbindungen mit bekannten, die Spaltreaktion von 1,2-Dichlorethan beschleunigenden Verbindungen verwendet werden.

2

Die erfindungsgemäß einzusetzenden Verbindungen werden zweckmäßig dem wie üblich gereinigten flüssigen 1,2-Di-chlorethan vor dessen Einspeisung in die thermische Zersetzung zugegeben. Zur besseren Dosierung empfiehlt es sich, von den Verbindungen zunächst eine konzentrierte, etwa 30 bis 60 gew.-%ige Lösung in gereinigtem 1,2-Di-chlorethan herzustellen und diese Lösung mit üblichen Dosierapparaten, zum Beispiel einer Dosierpumpe, in die Hauptmenge des gereinigten 1,2-Dichlorethans zu geben. Werden unter 0,001 Gew.-%, bezogen auf 1,2-Dichlorethan, von den erfindungsgemäß zuzusetzenden Verbindungen angewendet, so ist die beschleunigende Wirkung nicht mehr ausreichend. Bei einem Zusatz von über 5 Gew.-%, bezogen auf eingesetztes 1,2-Dichlorethan, überwiegen die Nachteile, beispielsweise vermehrte Nebenproduktbildung und höhere Kosten, den erzielbaren zusätzlichen Effekt. Vorzugsweise werden 0,005 bis 1 Gew.-%, bezogen auf eingesetztes 1,2-Dichlorethan, verwendet. Bei vergleichsweise niedrigeren Spalttemperaturen im Bereich von 350 bis etwa 450 °C wird man zweckmäßig Zusatzmengen wählen, die mehr im oberen Teil des Vorzugsbereiches, etwa zwischen 0,2 bis 1 Gew.-% liegen, während man bei höheren Spalttemperaturen, etwa im Bereich von 450 bis 550 °C, die Zusatzmengen zweckmäßig mehr im unteren Teil des Vorzugsbereiches, insbesondere von 0,005 bis 0,1 Gew.-%, bezogen auf eingesetztes 1,2-Dichlorethan, wählt.

Unter 300 °C Spalttemperatur führen die erfindungsgemäß einzusetzenden Verbindungen nicht mehr zu wirtschaftlich annehmbaren Umsätzen. Über 600 °C Spalttemperatur bringt das erfindungsgemäße Verfahren keine ausreichenden Vorteile mehr. Vorzugsweise wird im Temperaturbereich von 350 bis 550 °C gearbeitet.

Die mittlere Verweilzeit des gasförmigen 1,2-Dichlor-ethans in der auf Spalttemperatur erwärmten Zone kann im allgemeinen 2 bis 100 s betragen, wobei man zweckmäßig bei höheren Spalttemperaturen (500 bis 600 °C) niedrigere Verweilzeiten wählt als bei niedrigeren Spalttemperaturen (300 bis 500 °C). Bei Durchführung des neuen Verfahrens im technischen Maßstab wird man Verweilzeiten von 4 bis 40 s anwenden.

Das erfindungsgemäße Verfahren kann bei Normaldruck (0,0983 MPa) ausgeführt werden. Zur Steigerung der Raum-Zeit-Ausbeute im Spaltofen wird zweckmäßig bei Drücken bis zu etwa 5 MPa, vorzugsweise bei Drücken von 1 bis 4 MPa gearbeitet.

Die thermische Spaltung des 1,2-Dichlorethans mit dem erfindungsgemäßen Zusatz erfolgt nach üblichen Verfahren, beispielsweise in dem bekannten Röhrenspaltofen, in leeren Rohren von 20 bis 160 mm innerem Durchmesser, die von außen beheizt werden. Nach Verlassen der thermischen Spaltung werden die heißen Spaltgase wie üblich abgekühlt, der gebildete Chlorwasserstoff und das Vinylchlorid sowie das nichtumgesetzte 1,2-Dichlorethan destillativ abgetrennt und nach üblichen Verfahren gereinigt.

Das erfindungsgemäße Verfahren kann sowohl zur Senkung der Spalttemperatur bei gleichem Umsatz oder zur Erhöhung des Umsatzes bei gleicher Spalttemperatur verwendet werden. Insbesondere bei Senkung der Spalttemperatur wird neben der Energieersparnis eine Verminderung an störenden Nebenprodukten festgestellt. Das Verfahren kann mit geringem zusätzlichem apparativem Aufwand in den vorhandenen großtechnischen Spaltanlagen für 1,2-Dichlorethan durchgeführt werden.

Nachfolgende Beispiele und Vergleichsversuche sollen die Erfindung näher erläutern:
Vergleich verschiedener Initiatoren für die thermische Spaltung von 1,2-Dichlorethan.

Es wird eine Apparatur verwendet, bestehend aus einem Vorratsgefäß für 1,2-Dichlorethan, einem Rotameter, daran angeschlossen ein beheizbarer Glaskolben, der wiederum mit einem Quarzrohr verbunden ist, welches 18 mm Innendurchmesser hat und auf einer Länge von 500 mm beheizt werden kann. Der Ausgang des Quarzrohrs ist über eine Rückschlagsicherung mit 4 hintereinander geschalteten Waschflaschen und dahinter einer Kühlfalle verbunden. Die vom Ende des Quarzrohrs aus gesehen erste Waschflasche enthält destilliertes Wasser, die zweite eine 1 m wäßrige Lösung von Kaliumhydroxid, die dritte und vierte Waschflasche je 100 cm$^3$ Dimethylglykol. Die mit Wasser und die mit Kalilauge gefüllten Waschflaschen sind auf +5 °C gekühlt. Die Waschflaschen mit Dimethylglykol haben ca. 20 °C, die daran anschließende Kühlfalle ist auf -78 °C gekühlt.

Während des jeweiligen Versuchs wird der Glaskolben auf 350 °C und das Quarzrohr auf 400 °C (gemessen an der Rohraußenwand in der Mitte der Beheizungszone) erwärmt. Das Vorratsgefäß wird mit 1,2-Dichlorethan, dem jeweils 1 Gew.-% der in der nachfolgenden Tabelle angegebenen Initiatoren zugesetzt worden sind, gefüllt. Über das Rotameter laufen 47,2 g pro Stunde 1,2-Dichlorethan in den beheizten Glaskolben und werden dort verdampft. Die Dämpfe werden durch das Quarzrohr und die anschließenden Waschflaschen sowie die Kühlfalle geleitet. Nach einer Stunde wird der Versuch unterbrochen, das Dimethylglykol aus der dritten und vierten Waschflasche mit dem in der ersten und zweiten Waschflasche kondensierten 1,2-Dichlorethan vereinigt und mit Hilfe von quantitativer Gaschromatografie die in den vereinigten Flüssigkeiten enthaltene Vinylchloridmenge bestimmt. Gewichtsbilanz und Rücktitration der vorgelegten Kalilauge bestätigen das Resultat. Die erhaltenen Werte sind in Gewichtsprozent, bezogen auf die theoretisch bei 100 %-iger zur Ethanspaltung zu erwartende Vinylchloridmenge (= 100 Gew.-%) in nachfolgender Tabelle angegeben. In der Kühlfalle hatte sich kein Kondensat gebildet. Die Verweilzeit der 1,2-Dichlorethandämpfe in der beheizten Rohrzone (ohne Berücksichtigung des Anteils an gespaltenem 1,2-Dichlorethan berechnet) betrug etwa 17 s.

In allen nachfolgenden Tabellen sind die Vergleichsversuche gemäß Stand der Technik mit Buchstaben und die erfindungsgemäßen Beispiele mit Ziffern bezeichnet.

TABELLE I

Thermische Spaltung von 1,2-Dichlorethan mit je 1 Gew.-% Zusatz verschiedener Initiatoren bei 400 °C (Rohrwandtemperatur)

| Vergleichs-versuch bzw. Beispiel | Art des Zusatzes | Gew.-% Vinyl-chlorid nach der Spaltung | Bemer-kungen |
|---|---|---|---|
| A | kein Zusatz | 0,3 | nach Stand der Technik |
| B | Chlor ($Cl_2$) | 4 | |
| C | Thionylchlorid ($SOCl_2$) | 10,2 | |
| D | Sulfurylchlorid ($SO_2Cl_2$) | 24,8 | |
| E | Nitromethan ($CH_3NO_2$) | 6,5 | |
| F | Nitrosylchlorid ($NOCl$) | 32,7 | |
| G | Tetrachlormethan ($CCl_4$) | 2 | |
| H | Hexachlorethan ($C_2Cl_6$) | 6,5 | |
| 1 | Trichloracetylchlorid ($CCl_3COCl$) | 16 | erfindungsgemäß |
| 2 | Hexachlorpropen ($CCl_3CCl=CCl_2$) | 42 | |
| 3 | Heptachlorpropan ($CCl_3CCl_2CHCl_2$) | 62 | |
| 4 | Octachlorpropan ($C_3Cl_8$) | 89 | |

Vergleichsversuche I, K und L sowie Beispiele 5 und 6: Es wird folgende Apparatur verwendet: Ein Vorratsgefäß für 1,2-Dichlorethan ist über ein Rotameter mit einem heizbaren Glaskolben und dieser wiederum mit einen Quarzrohr von 6 mm Innendurchmesser verbunden, welches auf 500 mm Länge beheizt werden kann. Am Ende des Quarzrohres ist über eine Rückschlagsicherung eine Waschflasche angeschlossen, die eine 1 m wäßrige Lösung von Kaliumhydroxid mit einer Temperatur von 20 °C enthält. Der Ausgang der Waschflasche ist mit einem Gasometer verbunden. Zu Beginn der Versuche wird das Quarzrohr auf die jeweils in nachfolgender Tabelle II angegebenen Temperaturen (gemessen in der Mitte der Beheizungszone an der Rohraußenwand) erhitzt. Der Glaskolben ist auf 350 °C erwärmt. Das Vorratsgefäß wird mit 1,2-Dichlorethan gefüllt, das je nach Versuch die in nachfolgender Tabelle II angegebenen Initiatormengen enthält. Während 3 1/2 Stunden werden kontinuierlich 68 g des 1,2-Dichlorethans aus dem Vorratsgefäß über das Rotameter in den erwärmten Glaskolben gegeben, dort verdampft, in dem erhitzten Quarzrohr gespalten, anschließend mit Kalilauge gewaschen und die in der Wäsche nicht-kondensierten Gase im Gasometer gesammelt. Nach Beendigung des Versuches wird die im Gasometer vorhandene Gasmenge gemessen und ihre Zusammensetzung gaschromatografisch bestimmt. Ebenso wird das EDC-Kondensat gewogen und der quantitativen gaschromatografischen Analyse unterzogen. Aus diesen Daten wird der Umsatz des 1,2-Dichlorethans bestimmt.

Von einer Reihe Nebenprodukten wird die Fläche im Gaschromatogramm, bezogen auf die Gesamtfläche ( = 1 000 000),ermittelt, die so festgestellten Flächen-Tpm sind in nachfolgender Tabelle II angegeben. Der im Quarzrohr abgeschiedene Ruß wird durch übliche Verbrennungsanalyse bestimmt und ist in der Tabelle in mg (jeweils entstanden aus der unvollständigen thermischen Spaltung von 68 g 1,2-Dichlorethan) angegeben.

Als Initiatoren werden gemäß Stand der Technik Hexachlorethan ($C_2Cl_6$) und erfindungsgemäß Octachlorpropan ($C_3Cl_6$) verwendet.

**TABELLE II**

| Vergleichsversuch/Beispiel | I | K | 5 | L | 6 |
|---|---|---|---|---|---|
| Initiator-Art | - | $C_2Cl_6$ | $C_3Cl_8$ | $C_2Cl_6$ | $C_3Cl_8$ |
| Initiator-Menge (Gew.-%) | - | 0,01 | 0,01 | 1 | 1 |
| Spaltrohr-Wandtemperatur (°C) | 580 | 550 | 530 | 450 | 350 |
| 1,2-Dichlorethan-Umsatz (Gew.-%) | 53 | 52 | 52 | 56,5 | 58 |
| Nebenprodukte Flächen-Tpm — Ethan + Ethen | 129 | 106 | 105 | 61 | 59 |
| Propen | 11 | 8,2 | 15 | 4 | 3,9 |
| Ethin | 625 | 557 | 478 | 533 | 371 |
| Butadien (1,3) | 30 | 25 | 21 | 13 | 15 |
| Chlormethan | 45 | 45 | 19 | 27 | 20 |
| Buten(1)-in(3) | 48 | 33 | 29 | 33 | 12 |
| 1,1-Dichlorethen | 128 | 53 | 50 | 65 | 33 |
| Ruß (mg) | 2,22 | 1,01 | 0,93 | n.b. | n.b. |

(n.b. = nicht bestimmt)

**Patentansprüche**

1. Verfahren zur thermischen Spaltung von 1,2-Dichlor-ethan zu Vinylchlorid bei 300 bis 600 °C,unter Normaldruck oder erhöhtem Druck, in Gegenwart mindestens einer die Spaltreaktion beschleunigenden Verbindung, dadurch gekennzeichnet, daß als solche Verbindung(en) verwendet werden 0,001 bis 5 Gew.-%, bezogen auf eingesetztes 1,2-Dichlorethan, von Trichloracetylchlorid oder einer Verbindung, die 3 Kohlenstoffatome, mindestens 6 Chloratome, 0 oder 1 Sauerstoffatom und, je Kohlenstoffatom an dieses gebunden, 0 oder 1 Wasserstoffatom enthält.

2. Verfahren nach Anspruch 1, dadurch gekennzeichnet, daß in Gegenwart von 0,005 bis 1 Gew.-%, bezogen auf eingesetztes 1,2-Dichlorethan, von mindestens einer der in Anspruch 1 definierten, die Spaltreaktion beschleunigenden Verbindungen gearbeitet wird.

3. Verfahren nach einem der Ansprüche 1 oder 2, dadurch gekennzeichnet, daß in Gegenwart von Octachlorpropan, Heptachlorpropan oder Hexachlorpropen gearbeitet wird.

**Revendications**

1. Procédé de craquage thermique du dichloro-1,2 éthane en chlorure de vinyle, à une température de 300 à 600°C, sous la pression normale ou sous pression élevée, en présence d'au moins un composé accélérant la réaction de craquage, procédé caractérisé en ce qu'on utilise, comme composé(s) de ce genre, de 0,001 à 5% en poids, par rapport au dichloro-1,2 éthane mis en jeu, de chlorure de trichloracétyle ou d'un composé contenant trois atomes de carbone, au moins six atomes de chlore, 0 ou 1 atome d'oxygène et, sur chaque atome de carbone, 0 ou 1 atome d'hydrogène.

2. Procédé selon la revendication 1 caractérisé en ce qu'on opère en présence de 0,005 à 1% en poids, par rapport au dichloro-1,2 éthane mis en jeu, d'au moins un des composés accélérant la réaction de craquage qui ont été définis dans la revendication 1.

3. Procédé selon l'une des revendications 1 et 2 caractérisé en ce qu'on opère en présence d'octachloropropane, d'heptachloropropane ou d'hexachloropropène.

**Claims**

1. A process for the thermal cleavage of 1,2-dichloro-ethane to give vinyl chloride, at 300 to 600 °C, under atmospheric pressure or elevated pressure, in the presence of at least one compound accelerating the cleavage reaction, characterised by using as the compound(s) of this type 0.001 to 5 % by weight, relative to 1,2-dichloroethane used, of trichloroacetyl chloride or a compound which contains 3 carbon atoms, at least 6 chlorine atoms, 0 or 1 oxygen atom and, for each carbon atom bonded to the latter, 0 or 1 hydrogen atom.

2. The process as claimed in claim 1, characterised by carrying out said process in the presence of 0.005 to 1 % by weight, relative to 1,2-dichloroethane used, of at least one of the compounds, defined in claim 1, accelerating the cleavage reaction.

3. The process as claimed in one of claims 1 or 2, characterised by carrying out said process in the presence of octachloropropane, heptachloropropane, or hexachloropropene.